Europäisches Patentamt
European Patent Office
Office européen des brevets

(11) Veröffentlichungsnummer: **0 273 168**
**A1**

(12) **EUROPÄISCHE PATENTANMELDUNG**

(21) Anmeldenummer: **87116853.0**

(22) Anmeldetag: **14.11.87**

(51) Int. Cl.[4] **C07D 471/10**, A61K 31/445, //(C07D471/10,235:00,221:00)

(30) Priorität: **25.11.86 US 934756**

(43) Veröffentlichungstag der Anmeldung:
**06.07.88 Patentblatt 88/27**

(84) Benannte Vertragsstaaten:
**AT BE CH DE FR GB IT LI LU NL SE**

(71) Anmelder: **F. HOFFMANN-LA ROCHE & CO. Aktiengesellschaft**

**CH-4002 Basel(CH)**

(72) Erfinder: **Berger, Leo**
**7 Parkway**
**Montclair, N.J.(US)**
Erfinder: **Dairman, Wallace Mark**
**52 Benjamin Boulevard**
**Manahawkin, N.J. 08050(US)**
Erfinder: **Mowles, Thomas Francis**
**266 New Change Bridge Road**
**Pine Brook, N.J. 07058(US)**
Erfinder: **Olson, Gary Lee**
**431 Everson Place**
**Westfield, N.J.(US)**

(74) Vertreter: **Bertschinger, Christoph, Dr. et al**
**Grenzacherstrasse 124 P.O. Box 3255**
**CH-4002 Basel(CH)**

(54) **Triazaspirodecanylmethylindolon-Derivate.**

(57) Es werden Verbindungen der Formel

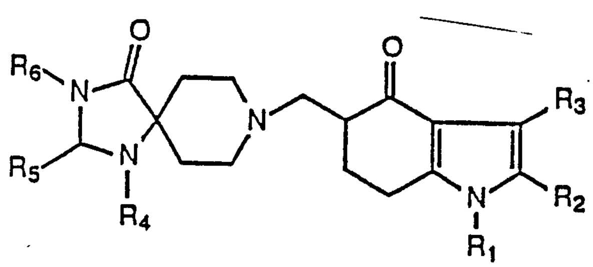

I

worin $R_1$ Wasserstoff, niederes Alkyl oder Acyl, $R_2$ und $R_3$ unabhängig voneinander je niederes Alkyl, Aralkyl oder Alkenyl, oder $R_2$ und $R_3$ zuammen einen Ring mit 5-8 Kohlenstoffatomen, $R_4$ Phenyl, substituiertes Phenyl, Cyclohexyl, substituiertes Cyclohexyl, Cyclopentyl, substituiertes Cyclopentyl, Cycloheptyl oder substituiertes Cycloheptyl, $R_5$ Wasserstoff oder niederes Alkyl und $R_6$ Wasserstoff, niederes Alkyl oder Acyl bedeuten,
und pharmazeutisch annehmbare Säureadditionssalze davon beschrieben. Die Verbindungen der Formel I

EP 0 273 168 A1

Xerox Copy Centre

und die pharmazeutisch annehmbaren Säureadditionssalze davon sind anti-emetische Wirkstoffe, welche keine Nebenwirkungen auf das Zentralnervensystem zeigen.

## Triazaspirodecanylmethylindolon-Derivate

Die Erfindung betrifft Verbindungen der Formel

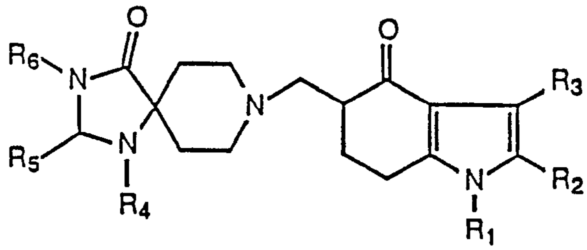

I

worin $R_1$ Wasserstoff, niederes Alkyl oder Acyl, $R_2$ und $R_3$ unabhängig voneinander je niederes Alkyl, Aralkyl oder Alkenyl, oder $R_2$ und $R_3$ zuammen einen Ring mit 5-8 Kohlenstoffatomen, $R_4$ Phenyl, substituiertes Phenyl, Cyclohexyl, substituiertes Cyclohexyl, Cyclopentyl, substituiertes Cyclopentyl, Cycloheptyl oder substituiertes Cycloheptyl, $R_5$ Wasserstoff oder niederes Alkyl und $R_6$ Wasserstoff, niederes Alkyl oder Acyl bedeuten,

und pharmazeutisch annehmbare Säureadditionssalze davon. Diese Verbindungen und Salze sind nützlich als anti-emetische Wirkstoffe.

Wie hierin verwendet, bezeichnet der Ausdruck "niederes Alkyl" einen gerad-oder verzweigtkettigen gesättigten Kohlenwasserstoffrest mit 1-7 Kohlenstoffatomen, z.B. Methyl, Aethyl, Propyl, Isopropyl, Butyl, t-Butyl, Neopentyl, Pentyl, Heptyl und dergleichen. Der Ausdruck "Alkenyl" bezeichnet einen gerad-oder verzweigtkettigen ungesättigten Kohlenwasserstoffrest mit 2-7 Kohlenstoffatomen, z.B. Vinyl, Allyl und dergleichen. Der Ausdruck "Aralkyl" bezeichnet eine durch eine Arylgruppe substituierte niedere Alkylgruppe, z.B. Phenyläthyl oder, vorzugsweise, Benzyl. Der Ausdruck "Aryl" bezeichnet Phenyl oder Phenyl, das durch einen oder mehrere Substituenten, ausgewählt aus der Gruppe bestehend aus Halogen, Trifluormethyl, niederem Alkyl, niederem Alkoxy, Nitro, Amino, niederem Alkylamino und di-niederem Alkylamino, substituiert ist. Der Ausdruck "niederes Alkoxy" bezeichnet eine Alkyläthergruppe, in welcher die niedere Alkylgruppe wie oben beschrieben ist, z.B. Methoxy, Aethoxy, Propoxy, Pentoxy und dergleichen. Der Ausdruck "Acyl" bezeichnet eine von einer aliphatischen Carbonsäure mit 1-7 Kohlenstoffatomen abgeleitete Alkanoylgruppe, z.B. Formyl, Acetyl, Propionyl und dergleichen. Der Ausdruck "Halogen" bezeichnet Brom, Chlor, Fluor und Jod. Der Ausdruck "niederer Alkohol" bezeichnet Alkohole mit 1-4 Kohlenstoffatomen, wie Methanol, Aethanol, Propanol und Butanol.

Wie hierin verwendet, bezeichnet der Ausdruck "substituiert" Substitution durch einen oder mehrere Substituenten, ausgewählt aus Chlor, Fluor, Brom, Jod, Hydroxy und niederes Alkyl.

Eine bevorzugte Gruppe von Verbindungen der Formel I sind diejenigen, worin $R_5$ und $R_6$ je Wasserstoff bedeuten.

Eine besonders bevorzugte Gruppe von Verbindungen der Formel I ist diejenige, worin $R_5$ und $R_6$ je Wasserstoff und $R_2$ und $R_3$ zusammen einen Ring mit 5 Kohlenstoffatomen und $R_4$ Phenyl bedeuten.

Bevorzugte Verbindungen der Formel I sind:

8-[(3-Aethyl-4,5,6,7-tetrahydro-2-methyl-4-oxo-1H-indol-5-yl)methyl]-1-phenyl-1,3,8-triazaspiro[4.5]-decan-4-on; und

8-[4,5,6,7-Tetrahydro-(2,3-dimethyl-4-oxo-1H-indol-5-yl)methyl]-1-phenyl-1,3,8-triazaspiro[4.5]decan-4-on.

Eine am stärksten bevorzugte Verbindung der Formel I ist:

1,2,3,5,6,7-Hexahydro-7-[(4-oxo-1-phenyl-1,3, 8-triazaspiro[4.5]decan-8-yl)methyl]-4H-cyclopent[b]indol-8-(8H)-on.

Beispiele von Verbindungen der Formel I sind:

1,2,3,5,6,7-Hexahydro-7-[(4-oxo-1-cycloheptyl-1,3,8-triazaspiro[4.5]decan-8-yl)methyl]-4H-cyclopent[b]indol-8(8H)-on;

1,2,3,5,6,7-Hexahydro-7-[4-oxo-1-butyl-1,3,8-triazaspiro[4.5]decan-8-yl)methyl]-4H-cyclopent[b]indol-8(8H)-on;

1,2,3,5,6,7-Hexahydro-7-[(4-oxo-1-methyl-1,3,8-triazaspiro[4.5]decan-8-yl)methyl]-4H-cyclopent[b]indol-8-(8H)-on;

1,2,3,5,6,7-Hexahydro-7-[(4-oxo-1-(4-chlorphenyl)-1,3,8-triazaspiro[4.5]decan-8-yl)methyl]-4H-cyclopent[b]-indol8(8H)-on;

1,2,3,5,6,7-Hexahydro-7-[(4-oxo-1-(4-methylphenyl)-1,3,8-triazaspiro[4.5]decan-8-yl)methyl]-4H-cyclopent[b]-indol-8(8H)-on;

1,2,3,5,6,7-Hexahydro-7-[(4-oxo-1-phenyl-1,3,8-triazaspiro[4.5]decan-8-yl)methyl]-4-methyl-4H-cyclopent[b]-indol-8(8H)-on;

1,2,3,5,6,7-Hexahydro-7-[(4-oxo-1-phenyl-1,3,8-triazaspiro[4.5]decan-8-yl)methyl]-4-acetyl-4H-cyclopent[b]-indol-8(8H)-on;

8-[(3-Aethyl-4,5,6,7-tetrahydro-2-(1-propenyl)-4-oxo-1H-indol-5-yl)methyl]-1-phenyl-1,3,8-triazaspiro[4.5]-decan-4-on;

8-[(3-Aethyl-4,5,6,7-tetrahydro-2-(1-phenylmethyl)-4-oxo-1H-indol-5-yl)methyl]-1-phenyl-1,3,8-triazaspiro[4.5]-decan-4-on;

8-[(3-Aethyl-4,5,6,7-tetrahydro-2-methyl-4-oxo-1H-indol-5-yl)methyl]-1-(4-methylcyclohexyl)-1,3,8-triazaspiro-[4.5]decan-4-on;

8-[(3-Aethyl-4,5,6,7-tetrahydro-2-methyl-4-oxo-1H-indol-5-yl)methyl]-1-phenyl-3-methyl-1,3,8-triazaspiro[4.5]-decan-4-on;

8-[(1,2,3,4,5,6,7,8-Octahydro-8-oxocyclopent[b]indol-7-yl)methyl]-2-methyl-1-phenyl-1,3,8-triazaspiro[4.5]-decan-4-on;

8-[(1,2,3,4,5,6,7,8-Octahydro-8-oxocyclopent[b]indol-7-yl)methyl]-1-cyclohexyl-1,3,8-triazaspiro[4.5]decan-4-on;

8-[(3-Aethyl-4,5,6,7-tetrahydro-2-methyl-4-oxo-1H-indol-5-yl)methyl]-1-cyclohexyl-1,3,8-triazaspiro[4.5]decan-4-on;

8-[4,5,6,7-Tetrahydro-(2,3-dimethyl-4-oxo-1H-indol-5-yl)methyl]-1-butyl-1,3,8-triazaspiro[4.5]decan-4-on;

8-[4,5,6,7-Tetrahydro-(2,3-dimethyl-4-oxo-1H-indol-5-yl)methyl]-1-cyclopentyl-1,3,8-triazaspiro[4.5]decan-4-on;

8-[4,5,6,7-Tetrahydro-(2,3-dimethyl-4-oxo-1H-indol-5-yl)methyl]-1(2-methylcyclopentyl)-1,3,8-triazaspiro[4.5]-decan-4-on; und

8-[(2,3,4,5,6,7,8,9-Octahydro-4-oxo-1H-carbazol-3-yl)methyl]-1-phenyl-1,3,8-triazaspiro[4.6]decan-4-on.

Erfindungsgemäss können die Verbindungen der Formel I und pharmazeutisch annehmbare Säureadditionssalze davon hergestellt werden, indem man eine Verbindung der allgemeinen Formel

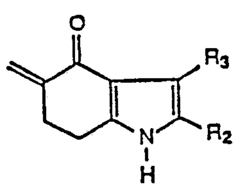

VII

worin $R_2$ und $R_3$ wie oben definiert sind, mit einer Verbindung der allgemeinen Formel

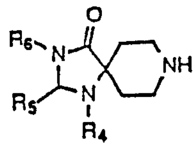

VIII

worin $R_4$, $R_5$ und $R_6$ wie oben definiert sind, zu einer Verbindung der Formel

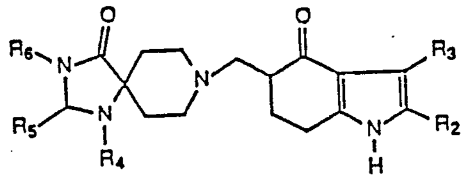

I'

worin $R_2$, $R_3$, $R_4$, $R_5$ und $R_6$ wie oben definiert sind,
umsetzt, erwünschtenfalls eine Verbindung der Formel I' einer Alkylierung oder Acylierung unterwirft und/oder erwünschtenfalls eine erhaltene freie Base in ein pharmazeutisch annehmbares Säureadditionssalz überführt.

Das erfindungsgemässe Verfahren und die Herstellung der Ausgangsstoffe werden durch das folgende Formelschema illustriert.

Formelschema

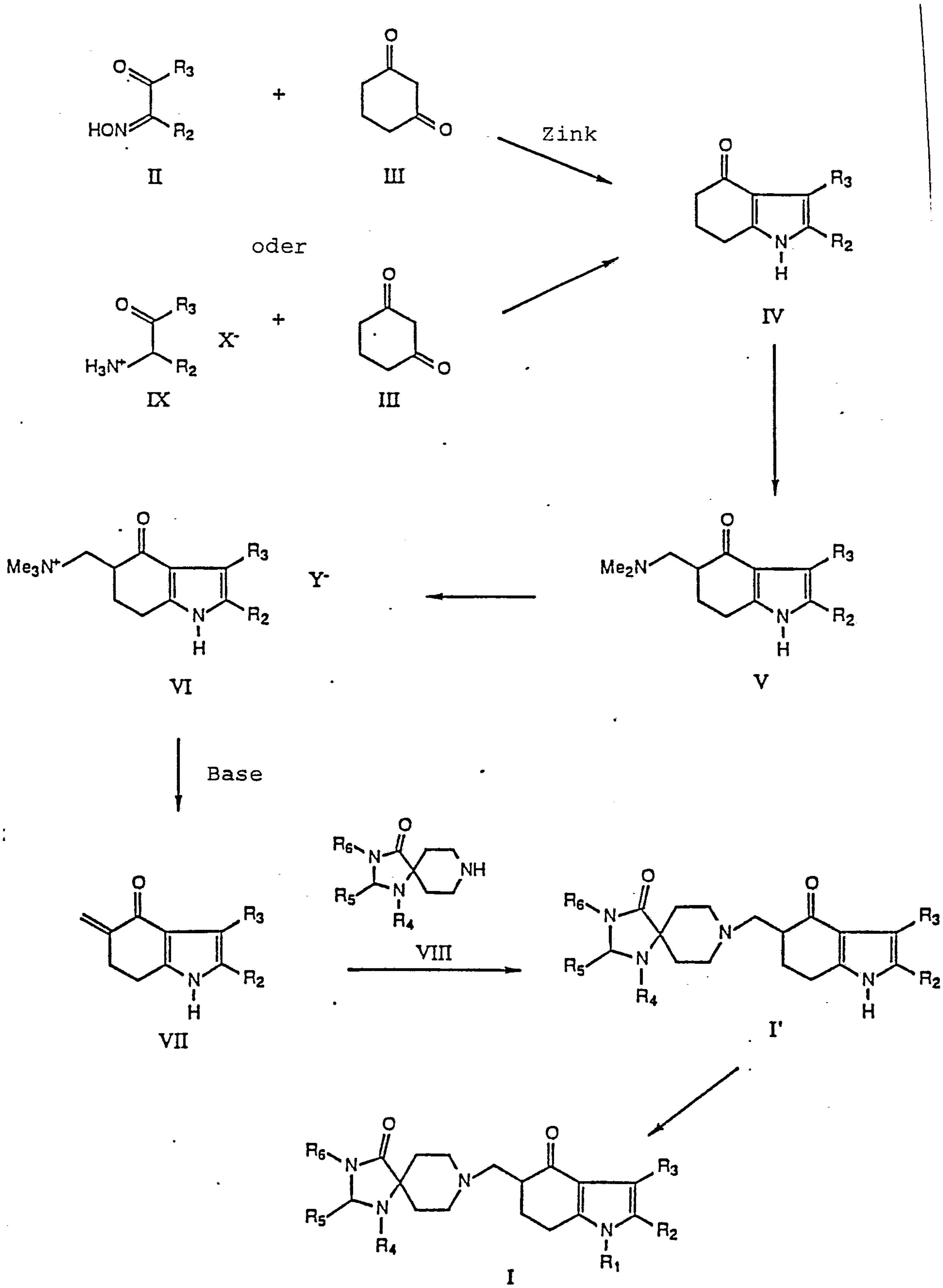

Die Reaktion einer Oximverbindung der Formel II, welche eine bekannte Verbindung ist oder nach an sich bekannten Methoden hergestellt werden kann, mit dem Diketon der Formel III, welches eine bekannte Verbindung ist, wird under Bedingungen durchgeführt, welche typisch sind für die Knorr-Reaktion. Diese Bedingungen umfassen die Verwendung eines Lösungsmittels wie 50-100-proz. wässrige Essigsäure, welche gegebenenfalls ein Kosolvens, wie einen niederen Alkohol, wie Methanol, enthalten kann. Das Reduktionsmittel in dieser Knorr-Pyrrol-Synthese ist Zinkstaub, das mindestens in stöchiometrischer Menge verwendet wird. Die Reaktion wird vorzugsweise bei einer Temperatur im Bereich von etwa 50°C bis zur Rückflusstemperatur des Lösungsmittels durchgeführt. Die Reaktion wird vorzugsweise unter Stickstoff oder Argon ausgeführt. Die erhaltene Verbindung der Formel IV wird mittels herkömmlicher Methoden isoliert, beispielsweise durch Extraktion oder Filtration, und wird durch Umkristallisieren oder Chromatographie gereinigt.

Als Alternative kann ein bekanntes Aminoketonsalz der Formel IX mit dem Diketon der Formel III in Abwesenheit eines Reduktionsmittels kondensiert werden, entweder im vorerwähnten Lösungsmittelsystem oder in einem wässrigen Lösungsmittel, das von etwa pH 3-5 gepuffert ist, aber nicht oberhalb pH 7.

Eine Verbindung der Formel IV kann durch eine Mannich-Reaktion mit Dimethylaminhydrochlorid und Formaldehyd in eine Verbindung der Formel V übergeführt werden. Im allgemeinen verwendet man einen Ueberschuss an Formaldehyd und Dimethylaminhydrochlorid. Jedes Lösungsmittel, das üblicherweise bei Mannich-Reaktionen verwendet wird, wie einen niederen Alkohol wie Aethanol, kann verwendet werden. Falls ein niederer Alkohol als Lösungsmittel verwendet wird, kann ein Nebenprodukt gebildet werden, das der Verbindung der Formel V entspricht, ausgenommen das ein Alkoxymethylensubstituent an Stelle von Wasserstoff am Pyrrolstickstoff atom gebunden ist. Dieses Nebenprodukt kann erwünschtenfalls durch Behandeln mit einer Säure, wie 2N Salzsäure, in die gewünschte Verbindung der Formel V übergeführt werden. Die Mannich-Reaktion wird im allgemeinen unter einer inerten Atmosphäre bei der Rückflusstemperatur des Lösungsmittels durchgeführt. Andere bekannte Methoden zur Durchführung der Mannich-Reaktion, beispielsweise mit einem tiefsiedenden Lösungsmittel unter Druck oder unter Verwendung eines forgeformten Methylenimmoniumsalzes, sind ebenfalls verwendbar.

Eine Verbindung der Formel V wird mit einem quaternisierenden Reagenz, wie Dimethylsulfat oder einem Alkylhalogenid, wie Methyljodid oder Methylchlorid oder, vorzugsweise, Methylbromid, umgesetzt. Falls Methylbromid verwendet wird, wird es als Gas in das Reaktionsgemisch eingeleitet. Die Quaternisierungsreaktion wird in einem aprotischen organischen Lösungsmittel, wie Methylenchlorid oder, vorzugsweise, Chloroform, durchgeführt, wobei man gelegentlich kühlt, um die Temperatur der Reaktionsmischung bei etwa Raumtemperatur zu halten.

Das erhaltene organische Salz der Formel VI kann durch Filtration isoliert und durch Umkristallisieren gereinigt werden falls erwünscht. Das Anion Y kann ein Halogenid, Methylsulfat oder dergleichen sein und hängt vom quaternisierenden Reagenz ab, das man bei der Herstellung des Salzes der Formel VI verwendet hat. Das Salz der Formel VI wird dann mit einer Base zu einer Methylenverbindung der Formel VII umgesetzt. Die verwendete Base ist im allgemeinen eine wässrige Base, wie verdünntes Natriumhyroxid, Kaliumhydroxid oder Bariumhydroxid. Die erhaltene Methylenverbindung wird durch herkömmliche Methoden, wie Filtration oder Extraktion, isoliert und durch Umkristallisieren oder Chromatographie gereinigt.

Eine Methylenverbindung der Formel VII wird mit einer äquimolaren Menge einer Piperidinverbindung der Formel VIII, welche eine bekannte Verbindung ist oder nach an sich bekannten Methoden hergestellt werden kann, zu einer Verbindung der Formel I', das ist eine Verbindung der Formel I, worin $R_1$ Wasserstoff bedeutet, umgesetzt. Die Reaktion wird in einem niederen Alkohol, wie Methanol, Propanol oder, vorzugsweise, Aethanol, bei einer Temperatur im Bereich von Raumtemperatur bis zur Rückflusstemperatur des Lösungsmittels, vorzugsweise bei der Rückflusstemperatur des Lösungsmittels, über einen Zeitraum von etwa eins bis etwa 36 Stunden durchgeführt. Die erhaltene Verbindung der Formel I' kann durch herkömmliche Methoden, wie Filtration oder Extraktion, isoliert und durch Umkristallisieren oder Chromatographie gereinigt werden.

Eine Verbindung der Formel I' kann erwünschtenfalls durch Alkylierung oder Acylierung in eine Verbindung der Formel I, worin $R_1$ niederes Alkyl oder Acyl bedeutet, übergeführt werden. In dieser Stufe wird das Anion einer Verbindung der Formel I' durch Behandlung mit einer starken Base in einem aprotischen Medium, wie Natriummethylsulfinylcarbanion in Dimethylsulfoxid, Lithiumdiisopropylamid in Tetrahydrofuran oder n-Butyllithium in Tetrahydrofuran, hergestellt. Das Anion wird dann mit einem Alkyl-oder Acylhalogenid behandelt, wobei man eine Verbindung der Formel I erhält, welche durch herkömmliche Methoden, beispielsweise durch Extraktion oder Filtration, isoliert und durch Umkristallisieren oder Chromatographie gereinigt wird. In dieser Stufe können Verbindungen der Formel I, worin $R_1$ und $R_6$ gleich sind,

durch Verwendung von zwei Aequivalenten der Base und zwei Aequivalenten des Alkylierungs-oder Acylierungsmittels hergestellt werden.

Wie oben ausgeführt, sind die Verbindungen der Formel VIII bekannte Verbindungen oder können nach an sich bekannten Methoden hergestellt werden.

Beispiele von Verbindungen der Formel VIII sind:

1-Phenyl-1,3,8-triazaspiro[4.5]-decan-4-on;
1-Cyclohexyl-1,3,8-triazaspiro[4.5]-decan-4-on;
1-Phenyl-2-methyl-1,3,8-triazaspiro[4.5]-decan-4-on;
1-Butyl-1,3,8-triazaspiro[4.5]-decan-4-on; und
1-(4-Chlorphenyl)-1,3,8-triazaspiro[4.5]-decan-4-on.

Wie oben ausgeführt, sind die Verbindungen der Formel II bekannte Verbindungen oder können nach an sich bekannten Methoden hergestellt werden. Im speziellen kann eine Verbindung der Formel II, worin $R_2$ und $R_3$ zusammen einen Ring mit 5-8 Kohlenstoffatomen bedeuten, durch Reaktion einer Verbindung der Formel X

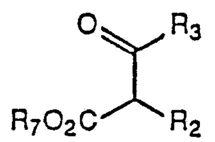

X

worin $R_2$ und $R_3$ wie gerade beschrieben sind, und $R_7$ Methyl oder Aethyl bedeutet, mit einer starken Base, wie wässriges Alkalimetall-oder Erdalkalimetallhydroxid, vorzugsweise Natriumhydroxid in Wasser, und Natriumnitrit bei einer Temperatur im Bereich von etwa -20°C bis 5°C, vorzugsweise bei 0°C, hergestellt werden. Die erhaltene Verbindung der Formel II kann durch Ansäuern und anschliessendem herkömmlichem Trennungsverfahren, wie Extraktion, abgetrennt und durch Umkristallisieren gereinigt werden.

Beispiele von Verbindungen der Formel II sind:

2-Hydroxyimino-3-pentanon;
2-Hydroxyimino-2-butanon; und
2-Hydroxyiminocyclopentanon.

Die Verbindungen der Formel I bilden Säureadditionssalze mit anorganischen oder organischen Säuren. Somit bilden sie pharmazeutisch annehmbare Säureadditionssalze sowohl mit pharmazeutisch annehmbaren organischen als auch anorganischen Säuren, beispielsweise mit Halogenwasserstoffsäuren, wie Salzsäure, Bromwasserstoffsäure, Jodwasserstoffsäure, und anderen Mineralsäuren, wie Schwefelsäure, Salpetersäure, Phosphorsäure oder dergleichen, Alkyl-und Monoarylsulfonsäuren, wie Aethansulfonsäure, Toluolsulfonsäure, Benzolsulfonsäure oder dergleichen, anderen organischen Säuren, wie Essigsäure, Weinsäure, Maleinsäure, Oxalsäure, Zitronensäure, Benzoesäure, Salicylsäure, Ascorbinsäure und dergleichen. Pharmazeutisch nicht annehmbare Säureadditionssalze von Verbindungen der Formel I können mittels herkömmlicher metathetischer Reaktionen in pharmazeutisch annehmbare Säureadditonssalze übergeführt werden, wobei das pharmazeutisch nicht annehmbare Anion durch ein pharmazeutisch annehmbares Anion ersetzt wird (oder alternativerweise durch Neutralisieren des pharmazeutisch nicht annehmbaren Säureadditionssalzes und Reaktion der so erhaltenen freien Base mit einem Reagenz, das ein pharmazeutisch annehmbares Säureadditionssalz liefert. Die Säureadditionssalze können auch Hydrate bilden.

Die Verbindungen der Formel I und ihre pharmazeutisch annehmbaren Säureadditionssalze entfalten wertvolle pharmakologische Aktivitäten als Dopaminrezeptorantagonisten. Sie sind deshalb nützlich als anti-emetische Wirkstoffe für die Behandlung von Nausea und Vomitus. Die Verbindungen der Formel I und ihre pharmazeutisch annehmbaren Säureadditionssalze wirken vorzugsweise an peripheren Rezeptorstellen, was im Vergleich zu bekannten anti-emetischen Wirkstoffen zu einer Verminderung der Nebenwirkungen auf das Zentralnerven system führt. Die Verbindungen der Formel I und ihre pharmazeutisch annehmbaren Säurenadditionssalze sind ebenfalls im Stande, die emetischen Reaktionen zu blockieren, welche durch eine Vielzahl von chemischen Agenzien, einschliesslich emetogenen Krebschemotherapeutika, wie Cisplatin, herorgerufen werden. Diese Wirkungen können durch die hiernach beschriebenen Verfahren demonstriert werden.

Der nachstehend beschriebene Spiroperidol-Bindungs-Test zeigt die Fähigkeit der Verbindungen der

Formel I, in vitro an Dopaminrezeptoren zu binden, was eine Eigenschaft von bekannten anti-emetischen Wirkstoffen ist.

Spiroperidol-Bindungs-Test

Dopaminrezeptor-Antagonismus durch Verbindungen der Formel I in vitro wird durch den $^3$H-Spiroperidol-Bindungs-Test ermittelt. In diesem Test wurden striäre Hirnhomogenate von Ratten während 20 Minuten in der Gegenwart von 0,2 nanomolarem $^3$H-Spiroperidol und unterschiedlichen Konzentrationen an Testverbindungen inkubiert. Die Inkubationen wurden durch rasche Filtration, gefolgt durch Waschen mit je 2×5 ml eiskaltem Test-Puffer (50mM Tribase-HCl pH 7,7, das in fixen Konzentrationen 0,1% Ascorbinsäure, 120mM NaCl, 5mM KCl, 2mM $CaCl_2$ und 1mM $MgCl_2$ enthält) beendet. Die Verbindungen der Formel I entfalteten die in der Tabelle I angegebenen Aktivitäten. Die $IC_{50}$ einer spezifischen Testverbindung ist diejenige Konzentration an Testverbindung, welche die Bindung von Spiroperidol an die Dopaminrezeptoren im Vergleich zu Kontrollen um 50% hemmt.

Tabelle I

$^3$H-Spiroperidol-Bindung In Vitro für Verbindungen der Formel I

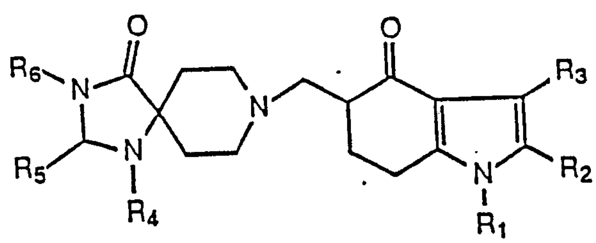

| Verb. | $R_1$ | $R_2$ | $R_3$ | $R_4$ | $R_5$ | $R_6$ | Bindung IC50 ,nM |
|---|---|---|---|---|---|---|---|
| Ia | H | $CH_3$ | $CH_2CH_3$ | Phenyl | H | H | 0.095 |
| Ib | H | -CH2CH2CH2- | | Phenyl | CH3 | H | 0.94 |
| Ic | H | -CH2CH2CH2- | | Cyclohexyl | H | H | 0.52 |
| Id | H | CH3 | CH3 | Phenyl | H | H | 0.078 |
| Ie | H | CH3 | CH2CH3 | Cyclohexyl | H | H | 0.082 |
| If | H | $-CH_2CH_2CH_2-$ | | Phenyl | H | H | 0.35 |

Wie durch die Tabelle I illustriert wird, sind die Verbindungen Ia-If allesamt potente Dopaminrezeptor-Antagonisten in vitro.

Weitere Testdaten zeigen die anti-emetische Wirkung der Verbindungen der Formel I in Tieren. Die Testbeschreibungen und Resultate sind wie folgt:

Anti-Apomorphin-und anti-D-Amphetamin-Tests in Mäusen

In Versuchstieren, welche zur Emesis fähig sind, ist Apomorphin wegen seiner dopaminagonistischen Aktivität an der Chemorezeptor-Trigger-Zone ein starkes Emetikum. Die Fähigkeit von dopaminantagonistischen Neuroleptika, die Apomorphin-induzierte Emesis in Hunden zu blockieren, korreliert mit ihrer antiemetischen Wirkung am Menschen, und solche Verbindungen sind klinisch wirksame Antiemetika. Weil klassiche Neuroleptika die Dopaminrezeptoren sowohl an der Chemorezeptor-Trigger-Zone als auch innerhalb des Zentralnervensystems blockieren, ist ihre Anwendung als Antiemetika mit einem häufigen Auftreten von Nebenwirkungen auf das Zentralnervensystem verbunden. Da die Chemorezeptor-Trigger-Zone funktionell ausserhalb der Blut-Hirn-Schranke liegt, wird ein Dopamin-Rezeptor-Antagonist mit antiemetischer Wirkung, welcher nicht in das Zentralnervensystem penetriert, keine Neigung zu Nebenwirkungen auf das Zentralnervensystem zeigen. Die Verbindungen der Formel I entfalten die bevorzugte Wirkung an Rezeptoren, welche ausserhalb der Blut-Hirn-Schranke sind, und zeigen eine weitegehende Trennung zwischen antiemetischer Wirkung und Nebenwirkungen auf das Zentralnervensystem.

Mäuse sind im Gegensatz zu Hunden oder Menschen nicht fähig zur Emesis. Allerdings reagieren Mäuse auf Apomorphin mit einem gut charakterisierten Aufbäumen. Dieses Apomorphin-induzierte Aufbäumen bei Mäusen entspricht einer emetischen Reaktion bei sensitiven Spezies und reflektiert eine Rezeptor-Besetzung in der Maus an einer Stelle, welche der Chemorezeptor-Trigger-Zone in Tieren, welche zur Emesis fähig sind, entspricht. Blockade der D-Amphetamin-Hyperaktivität reflektiert eine Blockade der Dopamin-Rezeptoren im Zentralnervensystem. Ein effektives Emetikum, das nur eine geringe Neigung zu Nebenwirkungen auf das Zentralnervensystem zeigt, sollte deshalb das Apomorphin-induzierte Aufbäumen bei niedrigeren Dosierungen blockieren als die D-Amphetamin-Hyperaktivität. Die Verfahren zur Ermittlung des anti-Apomorphin-Aufbäumens und der anti-Amphetamin-Hyperaktivität in Mäusen sind wie folgt:

Die Ermittlung der anti-Apomorphin-oder der anti-D-Amphetamin-Aktivität wurde an gefasteten männlichen Mäusen (47-54 Tage alt) durchgeführt.

Vorbehältlich anderer Angaben wurde die anti-Apomorphin-Aufbäum-Aktivität 2 Stunden nach der oralen Verabreichung der Test-Verbindung oder nach verschiedenen Zeitintervallen nach intravenöser Verabreichung der Testverbindung ermittelt. 1 mg/kg Apomorphin wurde intravenös verabreicht, wobei destilliertes Wasser als Vehikel verwendet wurde. Die Mäuse wurden dann in individuellen Maschendrahtkäfigen untergebracht. Während des Zeitraumes von 5-10 Minuten nach Verabreichung des Apomorphins wurden die Mäuse in Bezug auf das Aufbäumen beobachtet (mit den Vorderpfoten an der Seitenwand oder an der Decke des Käfigs und dem Kopf nach hinten gekrümmt). Mäuse, welche sich weniger als dreimal aufbäumten während dieses Zeitraumes, wurden vor dem Apomorphin für geschützt betrachtet.

Die anti-D-Amphetamin-Aktivität wurde wie folgt ermittelt: Gruppen von je 3 Mäusen pro Käfig wurden ungefähr 24 Stunden vor dem Testen in Plastikkäfigen untergebracht. Die Testverbindung wurde je 6 Mäusen pro Dosis oral oder intravenös verabreicht, und das Vehikel wurde 12 Mäusen verabreicht. Unmittelbar nach der Dosierung wurden die Mäuse in ihre ursprünglichen Käfige zurückgebracht, aus welchen man das Streu entfernt hatte. Die Käfige wurden dann auf elektronische Aktivitätsmesser gestellt. Nach Zeitintervallen von einer Stunde im Falle oral verabreichter Testverbindungen oder nach verschiedenen Intervallen im Falle intravenös verabreichter Testverbindungen erhielten die mit Testverbindung behandelten Mäuse und 6 der mit Vehikel behandelten Mäuse 10 mg/kg D-Amphetamin-Sulfat oral. Die verbleibenden 6 mit Vehikel behandelten Mäuse erhielten das Vehikel für D-Amphetamin-Sulfat, destilliertes Wasser oral. Ein konstantes Dosierungsvolumen von 0,2 ml/kg wurde verwendet. Nach der Dosierung wurden die Mäuse in ihre ursprünglichen Käfige zurückgebracht, und die motorische Aktivität wurde während der 10-70 Minuten nach der Dosierung überwacht. Insgesamt wurden 6 Aktivitätsmesser gleichzeitig verwendet. Jeder Messer hielt zwei Gruppen zu je 3 Mäusen, welche die Mäuse einer gegegenen Dosisgruppe, die Vehikel/D-Amphetamin-Sulfat-Gruppe oder die Vehikel/Vehikel-Gruppe umfasste. Die Hemmung der Hyperaktivität in % wurde berechnet, indem die auf dem Aktivitätsmesser gemessene Vehikel/D-Amphetamin-Aktivität als 0%-Hemmung und die auf dem Aktivitätsmesser gemessene Vehikel/Vehikel-Aktivität als 100% Hemmung festgelegt wurden.

Die $ED_{50}$ für Apomorphin-Aufbäumung ist diejenige Dosis, bei welcher 50% der behandelten Mäuse vor dem Apomorphin geschützt wurden. In den Fällen, in denen keine $ED_{50}$-Dosis ermittelt wurde, wurde der prozentuale Anteil der vor Apomorphin-Aufbäumung geschützten Tiere bei einer intravenösen Dosis von 10 mg/kg ermittelt. Die $ED_{50}$ für die D-Amphetamin-Hyperaktivität ist diejenige Dosis, bei welcher so behandelte Mäuse 50% Hemmung der Hyperaktivität zeigten.

Die Resultate für Verbindungen der Formel I im Apomorphin-Aufbäumungs-und D-Amphetamin-Hyperaktivität-Test, in welchen die Verbindungen der Formel I oral verabreicht worden sind, sind in der

nachfolgenden Tabelle II zusammengestellt. Die Testresultate in Tabelle II zeigten, dass die Verbindungen Id-Ie bei Verabreichung auf oralem Wege als Antagonisten der Apomorphin-Aufbäumung aktiv sind und im Amphetamin-Antagonismus-Test nur eine schwache Aktivität auf das Zentralnervensystem bewirken. Die Verbindungen Id-Ie zeigen somit ein Aktivitätsprofil, das zeigt, das sie bei Verabreichung auf dem oralen Weg als antiemetische Wirkstoffe nützlich sind.

## TABELLE II

Antagonismus der Apomorphin-Aufbäumung und der Amphetamin-Hyperaktivität für Verbindungen der Formel I und Vergleichsverbindungen, gegeben durch orale Verabreichung

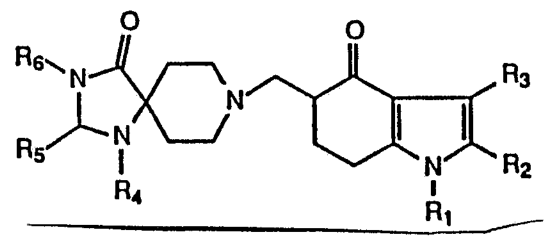

| Verb. | $R_1$ | $R_2$ | $R_3$ | $R_4$ | $R_5$ | $R_6$ | Antagonismus der Apomorphin-Aufbäumung $ED_{50}$, mg/kg | Antagonismus der Amphetamin-Hyperaktivität $ED_{50}$, mg/kg |
|---|---|---|---|---|---|---|---|---|
| Ia | H | $CH_3$ | $CH_2CH_3$ | Phenyl | H | H | >100 po | >100 po |
| Ib | H | $-CH_2CH_2CH_2-$ | | Phenyl | $CH_3$ | H | >100 po | >100 po |
| Ic | H | $-CH_2CH_2CH_2-$ | | Cyclohexyl | H | H | >100 po | >100 po |
| Id | H | $CH_3$ | $CH_3$ | Phenyl | H | H | 12.0 po | 147.0 po |
| Ie | H | $CH_3$ | $CH_2CH_3$ | Cyclohexyl | H | H | 98.0 po | 235.0 po |
| If | H | $-CH_2CH_2CH_2-$ | | Phenyl | H | H | 12.0 po | >100 po |
| <u>Vergleichsverbindungen</u> | | | | | | | | |
| Chlorpromazine-HCl | | | | | | | 4.9 po | 2.1 po |
| Haloperidol | | | | | | | 0.18 po | 0.23 po |
| Domperidone | | | | | | | 23.0 po | 194.0 po |

Die Resultate für Verbindungen der Formel I im Apomorphin-Aufbäumungs-und im D-Amphetamin-Hyperaktivitäts-Test, in welchen die Verbindungen der Formel I intravenös verabreicht worden sind, sind in der nachfolgenden Tabelle III zusammengestellt. Die Testresultate in Tabelle III zeigen, dass die Verbindungen Ia-Ie bei der Verabreichung auf dem intravenösen Wege aktiv sind als Antagonisten der Apomorphin-Aufbäumung und, soweit getestet, dass sie im Amphetamin-Antagonismus-Test nur eine schwache Aktivität auf das Zentralnervensystem bewirken. Die Verbindungen Ia-Ie besitzen somit ein Aktivitätsprofil, das zeigt, dass sie bei Verabreichung auf dem intravenösen Wege nützlich sind als anti-emetische Wirkstoffe.

## TABELLE III

Antagonismus der Apomorphin-Aufbäumung und D-Amphetamin-Hyperaktivität für Verbindungen der Formel I durch intravenöse Verabreichung

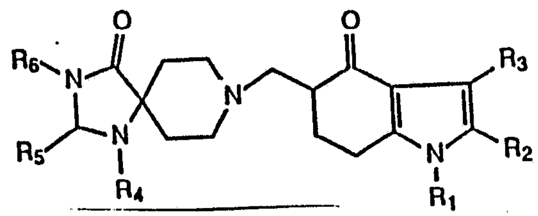

| Verb. | $R_1$ | $R_2$ | $R_3$ | $R_4$ | $R_5$ | $R_6$ | Antagonismus der Apomorphin-Aufbäumung: Zeit [a] (Std.) | Antagonismus der Apomorphin-Aufbäumung: $ED_{50}$ (mg/kg) | Antagonismus der Apomorphin-Aufbäumung: % geschützt bei 10 mg/kg (%) | Antagonismus der Amphetamin-Hyperaktivität: Zeit [a] (Std.) | Antagonismus der Amphetamin-Hyperaktivität: $ED_{50}$ (mg/kg) |
|---|---|---|---|---|---|---|---|---|---|---|---|
| Ia | H | $CH_3$ | $CH_2CH_3$ | Phenyl | H | H | 2 | 7.0 | - | 2 | 15.0 |
| | | | | | | | 7 | 2.3 | - | 7 | 8.6 |
| Ib | H | $-CH_2CH_2CH_2-$ | | Phenyl | $CH_3$ | H | 4 | - | 36 | - | - |
| | | | | | | | 7 | - | 36 | - | - |
| Ic | H | $-CH_2CH_2CH_2-$ | | Cyclohexyl | H | H | 4 | - | 40 | - | - |
| | | | | | | | 7 | - | 40 | - | - |
| Id | H | $CH_3$ | $CH_3$ | Phenyl | H | H | 4 | 5.1 | - | 4 | >20 |
| Ie | H | $CH_3$ | $CH_2CH_3$ | Cyclohexyl | H | H | 4 | - | 62 | - | - |
| | | | | | | | 7 | - | 79 | - | - |

a Zeit nach Verabreichung der Verbindung der Formel I

Zusätzliches Testen der erfindungsgemässen Verbindung If in anti-emetischen Tests wird unmittelbar hiernach beschrieben:

Anti-Apomorphin-Emesis in Hunden

Man verwendete weibliche Beagles mit einem ungefähren Körpergewicht von 15 kg. Für alle Hunde wurde vorher gezeigt, dass sie mindestens viermal erbrachen während der 60 Minuten nach Injektionen von 0,1 mg/kg Apomorphin•HCl s.c. in den Nacken. Jeder Hund wurde in Intervallen von 14 Tagen verwendet. Drei Sitzungen mit dem Medikament wurden gefolgt von einer Kontrollsitzung mit Apomorphin.

Die Verbindung If wurde, suspendiert in 5-proz. Gummi arabicum, in verschiedenen Dosierungen durch orale Gabe an mindestens 4 Hunden pro Dosis verabreicht. Eine Stunde später wurden 0,1 mg/kg Apomorphin•HCl s.c. injiziert, und die Hunde wurden für eine weitere Stunde auf Erbrechen hin beobachtet. Die anti-emetische Wirkung wurde ermittelt, indem man den Anteil derjenigen Tiere bestimmte, welche bei einer gegebenen Dosierung während der einstündigen Beobachtungsperiode nicht erbrachen. Die $ED_{50}$ ist diejenige Dosis, bei welcher 50% der Tiere bei einer gegebenen Dosierung während der einstündigen Beobachtungsperiode nicht erbrachen.

Die Verbindung If hatte in diesem Test eine anti-emetische $ED_{50}$ von 0,75 mg/kg oral. Die Vergleichsverbindung Domperidon, ein bekanntes Antiemetikum, hatte eine $ED_{50}$ von 0,2 mg/kg oral im gleichen Test.

Beurteilung der Nebenwirkungen an Hunden

Zur Beurteilung der Neigung zu Nebenwirkungen wurden Beagles verwendet. Vor der Substanzgabe wurden die Hunde während ungefähr 18 Stunden gefastet. Die Substanzgabe er folgte oral mittels Gelatinekapseln. Nach der Substanzgabe wurden die Hunde nach 15 und 30 Minuten und nach 1, 2, 3, 4, 5 und 6 Stunden auf abnormale Symptome hin beobachtet. Die Verbindung If wurde anfänglich oral in einer Dosis von 1 mg/kg verabreicht und dann einen Tag später in einer oralen Dosis von 10 mg/kg. Ein erfahrener Beobachter überwachte diese Hunde während 6 Stunden nach der Substanzgabe. Keine der Dosen verursachte irgend eine gut beobachtbare Veränderung im Verhalten oder in der motorischen Koordination, noch konnten irgendwelche andere abnormalen Symptome beobachtet werden.

Anti-Cisplatin-Emesis in Hunden

Die anti-emetische Wirkung der Verbindung If wurde ferner nachgewiesen, indem man dessen Aktivität beim Blockieren der Cisplatin-Emesis in Hunden nachwies. Das Verfahren war wie folgt:

Bei der Beurteilung wurden 6 männliche und 6 weibliche Beagles verwendet. Drei Hunde wurden täglich getestet. Jeder Hund erhielt entweder Kochsalz-Placebo, 30 ml i.v., die Verbindung If, 10 mg/kg i.v., oder Domperidon, 10/mg i.v. Die Verbindung If wurde in warmem, sterilem Wasser für Injektionszwecke gelöst. Domperidon wurde in Essigsäure gelöst, und der pH wurde mit 2N-NaOH eingestellt. Beide Substanzen wurden zu einem Infusionsvolumen von 30 ml mit sterilem Wasser verdünnt. Die Behandlung erfolgte durch Infusion über einen Zeitraum von 30 Minuten (n = 3) oder 60 Minuten (n = 9). 15 Minuten nach der Behandlung wurde Platinol® (Cisplatin für Injektionszwecke, Bristol Laboratories), 3 mg/kg i.v., verabreicht, und die emetische Aktivität wurde während 6 Stunden beobachtet. Das in sterilem Wasser gelöste Cisplatin, 1 mb/ml, wurde über einen Zeitraum von 10 Minuten infundiert. Jeder Behandlung wurde zwei männlichen und zwei weibliche, gewichtsmässig ausgegliche nen Hunden verabreicht.

Die Hunde wurden eine Stunde vor der Behandlung mit 20 g/kg in Wasser angefeuchtetem Hundefutter gefüttert. Während der Beobachtungsdauer wurden jeweils Beginn, Häufigkeit und Stärke der Vomitus-Episoden sowie jede atypische Verhaltensweise registriert.

Die statistische Auswertung der emetischen Aktivität wurde unter Verwendung des Student's-t-Test für ungepaarte Daten durchgeführt.

Wie durch die Tabelle IV illustriert wird, wurde mit der Verbindung If eine signifikante (ca. 66%)

Hemmung der Emesis im Vergleich zu Kontrollen beobachtet. Zum Vergleich, Domperidon unterschied sich nicht in signifikanter Weise von Kontrollen. Die Verbindung If unterscheidet sich somit von Domperidon, indem die Verbindung If wirksam ist sowohl gegen Apomorphin-als auch Cisplatin-Emesis in Hunden, wohingegen Domperidon nur gegen die Apomorphin-Emesis in Hunden wirksam ist.

TABELLE IV

CISPLATIN-INDUZIERTE EMESIS IN VIER BEAGLES

| BEHANDLUNG | DOSIS i.v. | VOMITUS-EPISODEN Mittel (+S.E.)6 Std. | VOMITUS-EPISODEN Mittel (+S.E.)1 Std. |
|---|---|---|---|
| Kochsalzlösung (nur Cisplatin) | 30ml | 19.8± 6.5 | 3.3± 1.0 |
| Verbindung If | 10mg/kg | 6.0± 1.8** | 1.0± 0.4* |
| Domperidon | 10mg/kg | 12.5± 2.3 | 2.1± 0.7 |

* $p \leq 0.05$ im Vergleich zu Cisplatin durch den ungepaarten Student's-t-Test.

** $p \leq 0.01$ im Vergleich zu Cisplatin durch den Mann--Whitney-Test.

$p \leq 0.057$ im Vergleich zu Domperidon.

Eine Verbindung der Formel I oder ein pharmazeutisch annehmbares Säureadditionssalz davon kann nach an sich bekannten Methoden verabreicht werden. Eine Verbindung der Formel I oder ein pharmazeutisch annehmbares Säureadditionssalz davon kann somit entweder allein oder mit anderen pharmazeutischen Wirkstoffen, z.B. chemotherapeutischen Wirkstoffen gegen Krebs, oral, parenteral oder rektal verabreicht werden. Für die orale Verabreichung kann eine Verbindung der Formel I in Form von Tabletten, Kapseln, z.B. in Mischung mit Talk, Stärke, Milchzucker oder anderen Bestandteilen, d.h. pharmazeutisch annehmbaren Trägern, oder in Form von wässrigen Lösungen, Suspensionen, Elixieren oder wässrigen alkoholischen Lösungen, z.B. in Mischungen mit Zucker und anderen Süssmitteln, Aromatisierungsmitteln, Färbemitteln, Verdickungsmitteln und anderen pharmazeutisch annehmbaren Hilfsstoffen, verabreicht werden. Für die parenterale Verabreichung kann eine Verbindung der Formel I in Lösungen oder Suspensionen, z.B. als wässrige oder Erdnussöl-Lösung oder Suspension unter Verwendun von Hilfsstoffen und Trägern, welche für diese Art der Verabreichung üblich sind, verabreicht werden.

Bei der Ausübung der vorliegenden Erfindung wird die Dosis einer Verbindung der Formel I oder eines pharmazeutisch annehmbaren Säureadditionssalzes davon von der Potenz und der Wirkungsdauer der im Einzelfall zu verabreichenden Verbindung der Formel I oder Salzes und vom Verabreichungswege, als auch von der Stärke der Krankheit, dem Alter des zu behandelnden Patienten und dergleichen abhängen. Dosen von Verbindungen der Formel I oder von pharmazeutisch annehmbaren Säureadditionssalzen davon, welche für die Ausübung der vorliegenden Erfindung ins Auge gefasst wurden, liegen im Bereich von etwa 0,35 mg/kg bis etwa 20 mg/kg täglich, vorzugweise etwa 0.75 mg/kg bis etwa 10 mg/kg täglich, entweder als Einzeldosis oder in mehrere Dosen aufgeteilt.

Die nachfolgenden Beispiele dienen der näheren Illustrierung der Erfindung. Alle Temperaturen sind, soweit nichts anderes erwähnt wird, in Celsiusgraden angegeben.

Beispiel 1

Herstellung von 1,2-Cyclopentandion-2-oxim

Eine Mischung von Aethyl-2-oxocyclopentancarboxylat (93,6 g, 0,60 Mol) und Natriumhydroxid (26,4 g, 0,66 Mol) in Wasser (550 ml) wurde während der Zugabe einer Lösung von Natriumnitrit (45.5 g, 0,66 Mol) in Wasser (150 ml) in einem Eisbad gerührt. Nach der Zugabe wurde die trübe Mischung bei Raumtemperatur während 48 Stunden gerührt, wobei sie klar wurde. Die Mischung wurde mit 20-proz. HCl auf pH 5 angesäuert und mit Methylenchlorid (5×300 ml) extrahiert. Die wässrige Phase wurde im Vakuum auf etwa ein Drittel des ursprünglichen Volumens konzentriert und erneut mit Methylenchlorid (2×100 ml) extrahiert. Die vereinigten Extrakte wurden mit Kochsalzlösung gewaschen, getrocknet ($Na_2SO_4$) und eingedampft, wobei man die Titelverbindung als tiefschmelzenden Festkörper erhält (44,4 g). Ein Teil wurde aus Petroläther umkristallisiert, wobei man kristalline Titelverbindung erhält, Schmelzpunkt 68-70°C; IR ($CHCl_3$) 3580, 3280, (OH), 1744 (C=O), 1640 $cm^{-1}$ (C=N); NMR ($CHCl_3$) 2.06 (m, 2, $CH_2$), 2.50 (t, 2, J=7 Hz, $CH_2$), 2.84 (t, 2, J=7 Hz), 9.1 (br s, 1, OH).

Beispiel 2

Herstellung von Hexahydro-4H-cyclopent[b]indol-8(8H)-on

Zu einer mechanisch gerührten Lösung von 1,2-Cyclopentandion-2-oxim (25,0 g, 0,22 Mol) und 1,3-Cyclohexandion (25,0 g, 0,22 Mol) in 70-proz. Essigsäure (250 ml) wurde Zinkstaub (43,0 g, 0,66 g-Atom) in Portionen zu etwa 5 g und über einen Zeitraum von 30 Minuten zugegeben (leicht exotherm). Nach der Zugabe wurde die Mischung während 2 Stunden unter Rückfluss erhitzt und dann auf Raumtemperatur abgekühlt. Die Lösung wurde vom überschüssigen Zink abdekantiert und in Eiswasser (250 ml) gegossen. Die Mischung wurde mit Methylenchlorid (1×200 ml, 2×100 ml) extrahiert, und die vereinigten Extrakte wurden mit gesättigtem Natriumbicarbonat bis zur Neutralität gewaschen, anschliessend mit Kochsalzlösung gewaschen und getrocknet ($Na_2SO_4$). Die Lösung wurde auf etwa ein Viertel des ursprünglichen Volumens konzentriert, worauf sich die Titelverbindung (8,3 g) als leicht gelbbraunen Festkörper abschied: Schmelzpunkt 236-238°C (aus Essigester umkristallisiert); IR (KBr) 3210 (NH), 1620 $cm^{-1}$ (C=O); NMR ($CHCl_3$) $\delta$ 2.07 (m, 2, $CH_2$), 2.30-2.90 (m, 10, $CH_2$), 10.17 (br s, 1, NH); MS m/e 175 ($M^+$). Anal. Ber. für $C_{11}H_{13}NO$: C, 75.40; H, 7.48; N, 7.99. Gefunden: C, 75.36; H. 7.38; N, 7.84.

Beispiel 3

Herstellung von 1,2,3,5,6,7-Hexahydro-7-(dimethylaminomethyl) -4H-cyclopent[b]indol-8(8H)-on

Eine Mischung von Hexahydro-4H-cyclopent[b]indol-8(8H)-on (7,0 g, 0.04 Mol), Dimethylaminhydrochlorid (4,88 g, 0,06 Mol) und Paraformaldehyd (1,8 g, 0.06 Mol) in Aethanol (140 ml) wurde während 18 Stunden zum Rückfluss erhitzt. Das Lösungsmittel wurde dann entfernt, und der Rückstand wurde an Kieselgel (trockene Kolonne) chromatographiert, wobei man mit der organischen Phase einer Mischung eluierte, welche durch Schütteln von 90 Teilen Chloroform, 30 Teilen Methanol, 10 Teilen Wasser und 6 Teilen Essigsäure (Volumen) hergestellt worden war. Die eluierten Fraktionen, welche das Produkt enthielten, wurden vereinigt, mit Bicarbonat gewaschen, mit Kochsalzlösung gewaschen, getrocknet ($Na_2SO_4$) und eingedampft, wobei man die Titelverbindung als weissen Festkörper (3,9 g) erhielt: Schmelzpunkt 164-165°C (aus Aethanol umkristallisiert); IR ($CHCl_3$) 3460, 3275 (NH) und 1620 $cm^{-1}$ (C=O) NMR ($CHCl_3$) 2.24 (s, 6, $N(CH_3)_2$) und 8.56 (br s, 1, NH); MS m/e 232 ($M^+$). Die Verbindung wurde mit HCl in Aethanol in das Hydrochloridsalz 5HCl übergeführt: Schmelzpunkt 207-208°C (aus Aethanol umkristallisiert). Anal. Ber. für $C_{14}H_{20}N_2O \bullet HCl$: C, 62.56; H, 7.88; N, 10.42. Gefunden: C, 62.14; H, 7.80; N, 10.30.

Beispiel 4

Herstellung von 1,2,3,5,6,7-Hexahydro-7-methylen-4H-cyclopent [b]indol-8(8H)-on

Methylbromid wurde in eine Lösung von 1,2,3,5,6,7-Hexahydro-7-(dimethylaminomethyl) -4H-cyclopent-[b]indol-8(8H)on (4,1 g, 17,5 mMol) in Chloroform (100 ml) eingeleitet, wobei man gelegentlich kühlte, um die Temperatur unterhalb 20°C zu halten, bis kein weiterer Niederschlag gebildet wurde (20 Minuten). Die weisse Suspension wurde während 20 Minuten gerührt und filtriert, wobei man 5,0 g des Methylbromidssalzes der Titelverbindung erhielt. Eine Portion von 3,5 g des quaternären Salzes (10,6 mMol) wurde in Wasser (30 ml) gelöst und bei 0-5°C mit 2N Natriumhydroxid (10,5 ml) behandelt. Die Mischung wurde während 30 Minuten gerührt, und der Niederschlag der Titelverbinung wurde abfiltriert, mit Wasser gewaschen und aus Aethanol umkristallisiert, wobei man die Titelverbindung (0,95 g) als weissen Festkörper erhielt: Schmelzpunkt 214-215°C: IR (KBr) 3205, 3160 (NH), 1648 (C=O), 1587 $cm^{-1}$ (C=C); NMR ($CHCl_3$) δ 2.40-2.80 (m,10, $CH_2$), 5.37 und 5.98 (2d, 2, $=CH_2$); 9.96 (br s, 1, NH); MS m e 187 (M ). Anal. Ber. für $C_{12}H_{13}NO$: C, 76.98; H, 7.00; N, 7.48. Gefunden: C, 76.70; H, 6.98; N, 7.45.

Beispiel 5

Herstellung von 1,2,3,5,6,7-Hexahydro-7-[(4-oxo-1-phenyl-1,3,8,triazaspiro [4,5]decan-8-yl)methyl]-4H-cyclopent[b]indol-8(8H)-on

Eine Mischung aus dem Endprodukt von Beispiel 4 (3,0 g, 16,0 mMol) und 1-Phenyl-1,3,8-triazaspiro-[4,5]-decan -4-on (3,7 g, 16,0 mMol) in Aethanol (90 ml) wurde während 24 Stunden zum Rückfluss erhitzt. Das Lösungsmittel wurde abgedampft, und der Rückstand wurde an Kieselgel (trockene Kolonne) chromatographiert, wobei man mit der organischen Phase eluierte, welche man durch Schütteln einer Mischung aus 90 Teilen $CHCl_3$, 30 Teilen $CH_3OH$, 10 Teilen $H_2O$ und 6 Teilen Essigsäure erhielt. Die Fraktionen wurden mit verdünnter $NH_4OH_4$ gewaschen, getrocknet ($Na_2SO_4$) und eingedampft. Kristallisation des chromatographierten Produktes aus Aethanol lieferte 3,35 g der Titelverbindung als weissen Festkörper: Schmelzpunkt 212-213°C; IR (KBr) 3240 (NH, OH), 1713 (C=O) und 1628 $cm^{-1}$ (C=O); NMR ($CHCl_3$ + DMSO) 1.50-3.10 (m, 21, $CH_2$),4.68 (s, 2, $CH_2$), 6.70-7.40 (m, 5, arom), 7.70 (s, 1, NH), und 9.77 (s, 1, NH); MS m/e 244, 187. Anal. Ber. für $C_{25}H_{30}N_4O_2$: C, 71.74; H, 7.23; N, 13.39. Gefunden: C, 71.49; H, 7.29; N, 13.18.

Behandlung der freien Base mit äthanolischer HCl lieferte das Hydrochloridsalz der Titelverbindung, Schmelzpunkt 252-253°C (Zers.). Anal. Ber. für $C_{25}H_{30}N_4O_2$•HCl: C, 65.99; H, 6.87; N, 12.31; Cl, 7.79. Gefunden: C, 65.60; H, 6.89; N, 12.18; Cl, 7.77.

Beispiel 6

Herstellung von 1,2,3,5,6,7-Hexahydro-7-[substituiertes Piperidinomethyl]-4H-cyclopent[b]-indol-8(8H)-on-Derivate oder anderen substituierten Piperidinomethylindolonen Allgemeines Verfahren

Eine Mischung von äquimolaren Mengen einer Verbindung der Formel VII und eines substituierten Piperidin-Derivates der Formel VIII in Aethanol wurde während 20-24 Stunden unter Rückfluss erhitzt. Das Lösungsmittel wurde abgedampft, und der Rückstand wurde an Kieselgel (trockene Kolonne) chromatographiert, wobei man mit der organischen Phase eluierte, welche durch Schütteln einer Mischung aus 90 Teilen $CHCl_3$, 30 Teilen $CH_3OH$, 10 Teilen $H_2O$ und 6 Teilen Essigsäure hergestellt wurde. Fraktionen wurden mit verdünnter $NH_4OH$ gewaschen, getrocknet ($Na_2SO_4$) und eingedampft. Der Rückstand wurde aus Aethanol kristallisiert und lieferte als Produkt das gewünschte substituierte 1,2,3,5,6,7-Hexahydro-7-[substituierte Piperidinylmethyl-4H-cyclopent[b]-indol-8(8H)-on-Derivat und andere substituierte Piperidinomethylindolone.

Dem allgemeinen Verfahren folgend, wurden die nachfolgend aufgeführten Analoga hergestellt.

A. Aus 1.9g 3-Aethyl-2-methyl-5-methylen-4,5,6,7-tetrahydro-1H-indol-4-on und 2.3g 1-Phenyl-1,3,8-triazaspiro[4.5]decan-4-on in Aethanol erhielt man 1.5g 8-[(3-Aethyl-4,5,6,7-tetrahydro-2-methyl-4-oxo1H-indol-5-yl)methyl]-1-phenyl-1,3,8-triazaspiro[4.5]decan -4-on, Smp. 205-7°C.

B. Aus 1.7g des Produktes aus Beispiel 4 und 2.23g 2-Methyl-1-phenyl-1,3,8-triazaspiro[4.5]decan-4-on in Aethanol erhielt man 1.3g 8-[(1,2,3,4,5,6,7,8--Octahydro-8-oxocyclopent[b]indol-7-yl)methyl]-2-methyl-1-phenyl-1,3,8-triazaspiro[4.5]decan-4-one, Smp. 234-6°C.

C. Aus 1.87g des Produktes aus Beispiel 4 und 2.37g 1-Cyclohexyl-1,3,8-triazaspiro[4.5]decan-4-on erhielt man 1.3g 8-[(1,2,3,4,5,6,7,8-Octahydro-8-oxocyclopent[b]indol-7-yl)methyl]-1-cyclohexyl-1,3,8-triazaspiro[4.5]decan-4-on, Smp. 211-4°C.

D. Aus 1.9g 3-Aethyl-2-methyl-5-methylen-4,5,6,7-tetrahydro-1H-indol-4-on und 2.37g 1-Cyclohexyl-1,3,8-triazaspiro[4.5]decan-4-on erhielt man 2.0g 8-[(3-Aethyl-4,5,6,7-tetrahydro-2-methyl-4oxo-1H-indol-5-yl)methyl]-1-cyclohexyl-1,3,8-triazaspiro[4.5]decan-4-on, Smp. 217-9°C.

E. Aus 1.75g 2,3-Dimethyl-5-methylen-4,5,6,7-tetrahydro-1H-indol-4-on und 2.3g 1-Phenyl-1,3,8--triazaspiro[4.5]decan-4-on erhielt man 1.14g 8-[4,5,6,7-Tetrahydro-(2,3-dimethyl-4-oxo-1H-indol-5-yl)methyl]-1-phenyl-1,3,8-triazaspiro[4.5]decan-4-on, Smp. 220-2°C.

Beispiel 7

Herstellung von 2-Hydroxyiminocyclopentanon

In einem mit einem mechanischen Rührer und einem Argoneinlass versehenen 3-l Kolben wurde eine Lösung von 70,5 g (1,76 Mol) Natriumhydroxid-Plätzchen in 1,0 l Wasser vorgelegt. Die Lösung wurde in einem Eisbad abgekühlt, und 250 g (1,60 Mol) Aethyl-2-cyclopentanon-carboxylat wurde in einer Portion dazugegeben. Die Mischung wurde, währenddem eine Lösung von 121,5 g (1,76 Mol) Natriumnitrit in 0,6 l Wasser über einen Zeitraum von etwa 20 Minuten tropfenweise dazugegeben wurde, gerührt. Nach der Zugabe wurde das Eisbad entfernt, und die Mischung wurde bei Raumtemperatur während 4 Tagen gerührt. Nach Ende dieses Zeitabschnittes wurde die Mischung mit 2×200ml Aether extrahiert. Die wässrige Phase wurde dann mit etwa 0,9 l 2N HCl angesäuert, bis pH 3 erreicht wurde. Die Mischung wurde dann mit 3×300 ml Aether extrahiert, mit Kochsalz gesättigt und mit zusätzlichen 5×300 ml Aether extrahiert. Die vereinigten Extrakte wurden über $Na_2SO_4$ getrocknet und auf einem Rotationsverdampfer bei 35°C eingedampft und lieferten als Produkt insgesamt 124,0 g (68,5%) 2-Hydroxyiminocyclopentanon als -schwach gelbes Oel, das beim Stehen in einer Gefrierkammer kristallisierte. Eine aus 30-60° Petroläther umkristallisierte Probe hatte einen Schmelzpunkt von 68-70°C.

Beispiel 8

Herstellung von Hexahydro-4H-cyclopent[b]indol-8(8H)-on

Zu einer Lösung von 27,6 g (0,25 Mol) 1,3-Cyclohexandion und 27,6 g (0,244 Mol) des rohen 2-Hydroxyiminocyclopentanones in 435 ml 70-proz. wässriger Essigsäure wurden 47,6 g (0,73 g-Atom) Zinkpulver in Portionen von etwa 5 g zugegeben. Die Reaktion verlief exotherm, aber es kam nicht zum Rückfluss. Die Mischung wurde unter Argon während 2 Stunden auf 80-85°C erhitzt und auf 1,3 l Eiswasser gegossen und mit 3×300 ml Methylenchlorid extrahiert. Die Methylenchlorid-Extrakte wurden bis zur Neutralität mit 3×500 ml gesättigter Natriumbicarbonatlösung gewaschen, über $Na_2SO_4$ getrocknet und auf einem Rotationsverdampfer bei 50°C konzentriert.

Nach Entfernen von etwa 75% des Lösungsmittels fiel die Titelverbindung aus der Lösung aus und wurde abfiltriert und mit Methylenchlorid gewaschen, um 9,6 g (22,4%) der Titelverbindung als weisslichen Festkörper zu liefern. Eine aus Essigester umkristallisierte Probe hatte einen Schmelzpunkt von 236-238°C.

Beispiel 9

Herstellung von 1,2,3,5,6,7-Hexahydro-7-[(dimethylamino)methyl] -4H-cyclopent[b]indol-8(8H)-on

Eine Lösung von 14,0 g (0,08 Mol) des Produktes aus Beispiel 8, 9,96 g (0,12 Mol) Dimethylamin-Hydrochlorid und 7,2 g (0,24 Mol) Paraformaldehyd in 280 ml Aethanol wurde unter mechanischem Rühren während 20 Stunden zum Rückfluss erhitzt. Das Lösungsmittel wurde in einem Rotationsverdampfer bei 35°C entfernt. Der Rückstand enthielt unverändertes Produkt aus Beispiel 8, die gewünschte Titelverbindung und eine dritte Komponente, welche durch Dünnschichtchromatographie als eine Aethoxymethyl-substituierte Verbindung identifiziert wurde. Der Rückstand wurde an Kieselgel (trockene Säule) chromatographiert, wobei mit der unteren Phase eluiert wurde, welche durch Schütteln einer Mischung aus 90 Teilen $CHCl_3$, 30 Teilen $CH_3OH$, 10 Teilen $H_2O$ und 6 Teilen Essigsäure hergestellt wurde. Fraktionen, welche das

Ausgangsketon und die Aethoxymethyl-substituierte Verbindung enthielten, wurden zuerst eluiert. Die Fraktionen, welche die Titelverbindung enthielten, wurden mit 10-proz. $NH_4OH$ gewaschen, über $Na_2SO_4$ getrocknet und in einem Rotationsverdampfer konzentriert und lieferten 6,7 g (36%) der Titelverbindung als weisslichen Festkörper. Eine aus Aethanol umkristallisierte Probe hatte einen Schmelzpunkt von 165-166°C.

Beispiel 9a

Herstellung von 1,2,3,5,6,7-Hexahydro-7-[(dimethylamino)methyl] -4H-cyclopent[b]indol-8(8H)-on

Andererseits kann die Titelverbindung auch gemäss dem Verfahren von Beispiel 9 mittels der Stufe des Abdampfens des Lösungsmittels auf einem Rotationsverdampfer hergestellt werden. Die erhaltene Reaktionsmischung kann mit 2N HCl be handelt werden, gefolgt von Extraktion des unveränderten Produktes aus Beispiel 8 mit Methylenchlorid. Die saure Lösung kann dann erwärmt werden, gefolgt von Basisstellen mit 2N NaOH, um die Titelverbindung auszufällen.

Beispiel 10

Herstellung von 1,2,3,5,6,7-Hexahydro-7-[(dimethylamino)methyl] -4H-cyclopent[b]indol-8(8H)-on-methylbromid-Salz

Eine Lösung von 20,7 g (0,089 Mol) der Titelverbindung von Beispiel 9 in 300 ml Chloroform wird in einem Eisbad abgekühlt, und Methylbromidgas wurde während 30 Minuten eingeleitet. Nach dem keine Entstehung von weiterem Niederschlag gesehen werden konnte, wurde die Mischung während einer Stunde bei 0°C gerührt und filtriert. Das feste Methylbromid-Salz wurde bei 25°C/1,0 mm über Nacht getrocknet und lieferte 27,2 g (93,3%) der Titelverbindung als weissen Festkörper.

Beispiel 11

Herstellung von 1,2,3,5,6,7,-Hexahydro-7-methylen-4H-cyclopent[b]indol -8(8H)-on

Zu einer mechanisch gerührten Suspension von 27,2 g (0,83 Mol) der rohen Titelverbindung von Beispiel 10 in 375 ml Wasser in einem Eisbad wurden 80 ml (1,6 Mol) 2N NaOH-Lösung via einem Tropftrichter dazugegeben. Nach Beendigung der Zugabe wurde die Mischung während einer Stunde im Eisbad gerührt und filtriert. Der Filterkuchen der Titelverbindung wurde in 500 ml 9:1 Methylenchlorid:Methanol gelöst. Die organische Phase wurde über $Na_2SO_4$ getrocknet und zu einem gelben Festkörper eingedampft. Der feuchte Festkörper wurde bei 25°C/1,0 mm getrocknet und lieferte 12,95 g (83,5%) der Titelverbindung. Eine aus Aethanol umkristalli sierte Probe hatte einen Schmelzpunkt von 214-215°C.

Beispiel 12

Herstellung von 1,2,3,5,6,7-Hexahydro-7-[(4-oxo-1-phenyl-1,3,8-triazospiro[4,5-]decan-8-yl)-methyl]-4H-cyclopent[b]indol-8(8H)-on-hydrochlorid

Eine Mischung aus 6,0 g (0,032 Mol) der Titelverbindung aus Beispiel 11 und 7,45 g (0,032 Mol) 1-Phenyl-1,3,8-triazaspiro[4,5]decan-4-on in 150 ml Aethanol wurde während 48 Stunden unter Argon zum Rückfluss erhitzt. Das Lösungsmittel wurde abgedampft und der Rückstand wurde an 500 g Kieselgel (trockene Kolonne) unter Eluieren mit der unteren Phase einer Mischung, welche durch Schütteln von 90 Teilen $CHCl_3$, 30 Teilen $CH_3OH$, 10 Teilen $H_2O$ und 6 Teilen Essigsäure erhalten worden war, chromatographiert. Die Fraktionen, welche die freie Base der Titelverbindung enthielten, wurden bei 35-50°C in einem Rotationsverdampfer eingedampft. Der Rückstand wurde in 500 ml Methylenchlorid gelöst und mit eiskaltem 1:1 $Na_4OH$:$H_2O$ auf pH 10-11 neutralisiert. Die Schichten wurden getrennt, und die organische Phase wurde über $Na_2SO_4$ getrocknet und konzentriert. Der verbleibende Festkörper wurde mit heissem Aethanol gewaschen und filtriert. Eine aus Aethanol umkristallisierte Probe der freien Base hatte einen Schmelzpunkt

von 183-185°C. Zur Herstellung des HCl-Salzes wurde der Filterkuchen in 100 ml Aethanol suspendiert und mit ungefähr 10 ml 5N HCl in Aether behandelt. Die Lösung wurde konzentriert und abkühlen gelassen, wobei das Hydrochlorid-Salz der Titelverbindung ausfiel. Zwei Portionen wurden erhalten, welche nach Trocknen über Nacht bei 100°C 1,0 mm insgesamt 5,85 g des Hydrochlorid-Salzes der Titelverbindung lieferten.

In den Formulierungsbeispielen sind die Verbindungen wie folgt:

Verbindung If ist 1,2,3,5,6,7-Hexahydro-7-[(4-oxo-1-phenyl-1,3,8-triazaspiro[4.5]decan-8-yl)methyl]-4H-cyclopent[b]indol-8(8H)-on.

Verbindung Ia ist 8-[(3-Aethyl-4,5,6,7-tetrahydro-2-methyl-4-oxo-1H-indol-5-yl)methyl]-1-phenyl-1,3,8-triazaspiro[4.5]-decan-4-on.

Verbindung Ib ist 8-[(1,2,3,4,5,6,7,8-Oxtahydro-8-oxocyclopent[b]indol-7-yl)methyl]-2-methyl-1-phenyl-1,3,8-triazaspiro[4.5]-decan-4-on.

Verbindung Ic ist 8-[(1,2,3,4,5,6,7,8,-Octahydro-8-oxocyclopent[b]indol-7-yl)methyl]-1-cyclohexyl-1,3,8-triazaspiro[4.5]-decan-4-on.

Verbindung Id ist 8-[4,5,6,7-Tetrahydro-(2,3-dimethyl-4-oxo-1H-indol-5-yl)methyl]-1-phenyl-1,3,8-triazaspiro[4.5]decan-4-on.

Verbindung Ie ist 8-[(3-Aethyl-4,5,6,7-tetrahydro-2-methyl-4-oxo-1H-indol-5-yl)methyl]-1-cyclohexyl-1,3,8-triazaspiro[4.5]decan-4-on.

Beispiel 13

## PARENTERALE FORMULIERUNG
## (für intravenöse und intramuskuläre Verwendung)

| | BESTANDTEILE | mg/ml | |
|---|---|---|---|
| 1. | Verbindung If* | 2.0mg | 10.0mg |
| 2. | Propylenglycol** | 100 mg | 500 mg** |
| 3. | Emulphor** | 10 mg | 50 mg** |
| 4. | Wasser für Injektionszwecke q.s. ad | 1 ml | 1 ml |

Verfahren zur Herstellung:

1) Löse Bestandteile 1 und 2 in Wasser für Injektionszwecke.
2) Gib Bestandteil 1 dazu und löse oder suspendiere in der Lösung aus Stufe 1.
3) Justiere den pH unter Verwendung von verdünntem Natriumhydroxid oder Salzsäure. ***
4) Gib Wasser für Injektionszwecke in gewünschter Menge dazu.
5) Fülle die Lösung in geeignete Behälter ab. * Formulierung kann auch auf Verbindungen Ia, Ib, Ic, Id und Ie angewendet werden.

**Lösungsmittel oder Lösungsvermittler, wie Polyäthylenglykol, Alkohol, Dimethylacetamid, Glyzerin, Povidon, Lezithin, Sorbitan-Monooleat und -Trioleat, Polysorbat 20 oder 80 können allein oder in Kombination verwendet werden, um eine genügende Löslichkeit und Stabilität zu erzielen.

***Puffer, wie Citrat, Acetat oder Phosphat, kö nnen zwecks genü gender Stabilisierung eingearbeitet werden.

Beispiel 14

TABLETTENFORMULIERUNG (Trockengranulierung)

| | BESTANDTEILE | mg/Tabletten | |
|---|---|---|---|
| 1. | Verbindung If* | 2mg | 20mg |
| 2. | Stärke | 20mg | 40mg |
| 3. | Avicel | 40mg | 80mg |
| 4. | Lactose | 137mg | 274mg |
| 5. | Magnesiumsterat | 1mg | 2mg |
| | | 200mg | 400mg |

Verfahren zur Herstellung:

1) Mische Bestandteile 3 und 4 in einem geeigneten Mixer.
2) Füge hinzu und mische die Verbindung 1f zur Mischung aus Stufe 1.
3) Füge hinzu und mische Bestandteil 2 zur Mischung aus Stufe 2.
4) Füge hinzu und mische Bestandteil 5 zur Mischung aus Stufe 3.
5) Verpresse das Granulat auf einer geeigneten Tablettenpresse. * Formulierung kann auch auf die Verbindungen Ia, Ib, Ic, Id und Ie angewendet werden.

**Ansprüche**

1. Eine Verbindung der Formel

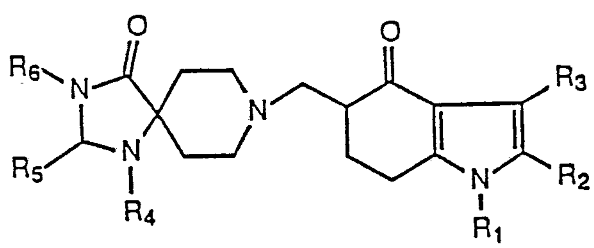

I

worin $R_1$ Wasserstoff, niederes Alkyl oder Acyl, $R_2$ und $R_3$ unabhängig voneinander je niederes Alkyl, Aralkyl oder Alkenyl, oder $R_2$ und $R_3$ zuammen einen Ring mit 5-8 Kohlenstoffatomen, $R_4$ Phenyl, substituiertes Phenyl, Cyclohexyl, substituiertes Cyclohexyl, Cyclopentyl, substituiertes Cyclopentyl, Cycloheptyl oder substituiertes Cycloheptyl, $R_5$ Wasserstoff oder niederes Alkyl und $R_6$ Wasserstoff, niederes Alkyl oder Acyl bedeuten,
oder ein pharmazeutisch annehmbares Säureadditionssalz davon.

2. Verbindungen nach Anspruch 1, worin $R_5$ und $R_6$ je Wasserstoff bedeuten.

3. Verbindungen nach Anspruch 2, worin $R_2$ und $R_3$ zusammen einen Ring mit 5 Kohlenstoffatomen bilden, und $R_4$ Phenyl bedeutet.

4. 1,2,3,5,6,7-Hexahydro-7-[(4-oxo-1-phenyl-1,3,8-triazaspiro[4.5]decan-8-yl)methyl]-4H-cyclopent[b]-indol-8(8H)-on, oder ein pharmazeutisch annehmbares Säureadditionssalz davon.

5. 8-[(3-Aethyl-4,5,6,7-tetrahydro-2-methyl-4-oxo-1H-indol-5-yl)methyl]-1-phenyl-1,3,8-triazaspiro[4.5]-decan-4-on oder ein pharmazeutisch annehmbares Säureadditionssalz davon.

6. 8-[4.5.6.7-Tetrahydro-(2.3-dimethyl-4-oxo-1H-indol-5-yl)methyl]-1-phenyl-1.3.8-triazaspiro[4.5]-decan-4-on. oder ein pharmazeutisch annehmbares Säureadditionssalz davon.

7. Verbindungen nach einem der Ansprüche 1-6 zur Verwendung als therapeutische Wirkstoffe.

8. Verbindungen nach einem der Ansprüche 1-6 zur Verwendung als anti-emetische Wirkstoffe.

9. Ein Verfahren zur Herstellung von Verbindungen nach einem der Ansprüche 1-6, dadurch gekennzeichnet, dass man eine Verbindung der allgemeinen Formel

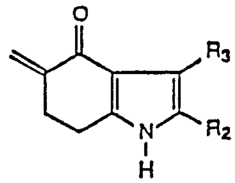

VII

worin $R_2$ und $R_3$ wie in Anspruch 1 definiert sind, mit einer Verbindung der allgemeinen Formel

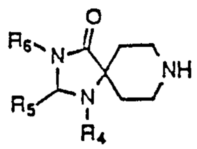

VIII

worin $R_4$, $R_5$ und $R_6$ wie in Anspruch 1 definiert sind,
zu einer Verbindung der Formel

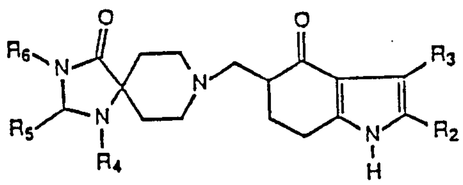

I'

worin $R_2$, $R_3$, $R_4$, $R_5$ und $R_6$ wie in Anspruch 1 definiert sind,
umsetzt, erwünschtenfalls eine Verbindung der Formel I' einer Alkylierung oder Acylierung unterwirft und oder erwünschtenfalls eine erhaltene freie Base in ein pharmazeutisch annehmbares Säureadditionssalz überführt.

10. Ein pharmazeutisches Präparat enthaltend eine Verbindung nach einem der Ansprüche 1-6 und einen therapeutisch inerten Träger.

11. Ein anti-emetisch wirksames Präparat enthaltend eine Verbindung nach einem der Ansprüche 1-6 und einen therapeutisch inerten Träger.

12. Verwendung von Verbindungen nach einem der Ansprüche 1-6 bei der Behandlung oder Verhütung von Krankheiten.

13. Verwendung von Verbindungen nach einem der Ansprüche 1-6 bei der Behandlung oder Verhütung von Nausea und Vomitus.

14. Verwendung von Verbindungen nach einem der Ansprüche 1-6 zur Herstellung von anti-emetisch wirksamen pharmazeutischen Präparaten.

Europäisches Patentamt

# EUROPÄISCHER RECHERCHENBERICHT

Nummer der Anmeldung

EP 87 11 6853

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-1 950 072 (ALDRICH CHEMICAL CO. INC.)<br>* Ansprüche 1, 6-15; Seite 1 *<br>--- | 1,2,7-14 | C 07 D 471/10<br>A 61 K 31/445//<br>(C 07 D 471/10<br>C 07 D 235:00<br>C 07 D 221:00 ) |
| Y | DE-A-2 630 602 (SQUIBB & SONS, INC.)<br>* Ansprüche 1, 2, 15, 16, 20; Seite 2, letzter Absatz - Seite 3, Zeile 2 *<br>--- | 1,2,7-14 | |
| A | EP-A-0 091 510 (HOECHST-ROUSSEL PHARMACEUTICALS INC.)<br>* Ansprüche 1, 2, 4-6 *<br>--- | 1,2,7,9,10,12 | |
| A | GB-A-1 108 578 (ENDO LABORATORIES INC.)<br>* Ansprüche 1, 2, 16, 18 *<br>--- | 1,2,7,9,10,12 | |
| A | US-A-3 845 060 (HUEBNER)<br>* Zusammenfassung *<br>--- | 1,2,7,10,12 | |
| A | US-A-4 526 896 (SCHERRER et al.)<br>* Beispiel 11 *<br>----- | 1,2 | **RECHERCHIERTE SACHGEBIETE (Int. Cl.4)**<br>C 07 D 209/00<br>C 07 D 471/00 |

Der vorliegende Recherchenbericht wurde für alle Patentansprüche erstellt

| Recherchenort | Abschlußdatum der Recherche | Prüfer |
|---|---|---|
| BERLIN | 29-02-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)